# EUROPÄISCHE PATENTSCHRIFT

(11) **EP 1 752 191 B1**
(45) Veröffentlichungstag und Bekanntmachung des Hinweises auf die Patenterteilung: **10.09.2008**
(21) Anmeldenummer: 06014806.1
(22) Anmeldetag: 17.07.2006
(51) Int. Cl.: A61Q 5/08, A61Q 5/10, A61K 8/11

(54) **Mittel zum Aufhellen und/oder Färben von keratinhaltigen Fasern mit beschichtetem Alkalisierungsmittel**
Agent for bleaching and/or colouring keratinic fibres with a coated alkaline agent
Agent à eclairir et/ou colorer des fibres kératiniques avec un agent d'alcalinisation enrobé

(30) Priorität: 10.08.2005 DE 102005038073
(43) Veröffentlichungstag der Anmeldung: 14.02.2007
(73) Patentinhaber: Henkel AG & Co. KGaA, 40589 Düsseldorf (DE)
(72) Erfinder: Höffkes, Horst, 40595 Düsseldorf (DE); Bossmann, Britta, 40699 Erkrath (DE)

(56) Entgegenhaltungen:
- EP-A2- 0 583 767
- WO-A-20/06018099
- GB-A- 1 275 173
- US-A- 3 726 967

## Beschreibung

Die Erfindung betrifft Mittel zum Aufhellen und/oder Färben von keratinhaltigen Fasern, insbesondere Blondiermittel für menschliche Haare.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepaßt werden. Dabei können Dauerwell- und andere die Haarform verändernde Verfahren nahezu unabhängig vom Typ der zu behandelnden Haare eingesetzt werden. Dagegen sind Färbe- und insbesondere Blondierverfahren auf bestimmte Ausgangshaarfarben begrenzt. So eignen sich für aufhellende Verfahren, die sogenannten Blondierverfahren, im wesentlichen nur dunkelblonde oder hellere Haare. Die Grundlagen der Blondierverfahren sind dem Fachmann bekannt und können in einschlägigen Monographien, z.B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, nachgelesen werden.

So werden üblicherweise feste oder pastenförmige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Die genannten Zubereitungen, die vor der Anwendung mit einer Wasserstoffperoxidlösung vermischt werden, werden im weiteren als "Blondiermittel" bzw. "Blondierpulver" bezeichnet. Alle aufgeführten Mengenangaben beziehen sich, soweit nicht anders ausgeführt, ausschließlich auf diese Zubereitungen.

Die vorliegende Erfindung bezieht sich auch auf Mittel zum gleichzeitigen Färben und Aufhellen von keratinhaltigen Fasern, insbesondere menschlichen Haaren. Konventionelle Haarfärbemittel bestehen in der Regel aus mindestens einer Entwickler- und mindestens einer Kupplersubstanz und enthalten ggf. noch direktziehende Farbstoffe als Nuanceure. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.
Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Vor ihrer Anwendung auf menschliches Haar werden Haarfärbemittel mit Oxidationsfarbstoffvorprodukten mit verdünnter wäßriger Wasserstoffperoxid-Lösung vermischt. Die Einwirkungsdauer auf dem Haar zur Erzielung einer vollständigen Ausfärbung liegt zwischen etwa 30 und 40 Minuten. Es ist naheliegend, daß bei den Benutzern dieser Haarfarben ein Bedürfnis besteht, diese Einwirkungszeit zu verringern.

Gleichzeitig besteht auch vielfach der Wunsch, zusammen mit der Haarfärbung auch eine Aufhellung des zu färbenden Haares, die sich auch als erhöhter Glanz bemerkbar macht, zu erreichen.

Sowohl in reinen Blondiermitteln als auch in aufhellenden Färbemitteln besteht das Problem, daß ein Oxidationsmittel, das dem Mittel zugesetzt wird, um ein Anwendungsprodukt zu erhalten, schnell zersetzt wird. Diese Zersetzungsreaktion wird insbesondere durch Alkalisierungsmittel beschleunigt. Bei Zersetzung von Wasserstoffperoxid tritt eine Gasentwicklung ein. Diese kann während der Handhabung der Blondiermittel je nach Intensität ein Gefahrenpotenzial für den Anwender in sich bergen.

Der vorliegenden Erfindung lag die Aufgabe zugrunde, Mittel zum Aufhellen und/oder Färben von Keratinfasern bereitzustellen, die eine bessere Stabilität von Oxidationsmitteln, die dem Mittel vor der Anwendung zugesetzt werden, ermöglichen. Insbesondere Wasserstoffperoxid sollte dabei stabilisiert werden, ohne daß auf den Einsatz von Alkalisierungsmitteln verzichtet werden muß.

In einer ersten Ausführungsform betrifft die vorliegende Erfindung Mittel zum Aufhellen und/oder Färben von Keratinfasern, insbesondere menschlichen Haaren, enthaltend mindestens ein festes Alkalisierungsmittel, das einen Partikelkern umfaßt, der Alkalisierungsmittel enthält und eine diesen Kern umgebende Hülle aufweist, die zu mindestens 50 Gew.-%, vorzugsweise zu mindestens 70 Gew.-%, weiter bevorzugt zu mindestens 90 Gew.-% und insbesondere zu 100 Gew.-% ihres Gewichts aus Fettsäuren ausgewählt aus Caprylsäure, Pelargonsäure, Caprinsäure, Undecansäure, Laurinsäure, Tridecansaure, Tetradecansäure, Pentadecansäure, Palmitinsäure, Margarinsäure, Stearinsäure, Isostearinsäure, Nonadecansäure, Arachinsäure, Behensäure, Lignocerinsäure, Cerotinsäure, Melissinsäure, Crotonsäure, Ölsäure, Erucasäure, Sorbinsäure, Elaeostearinsäure, Docosahexaensäure besteht.

Nachfolgend werden die erfindungsgemäßen Mittel zum Aufhellen und/oder Färben von Keratinfasern auch als "Blondier- bzw. Aufhell- und Färbemittel", als "Blondiermittel", als "Aufhellmittel" oder als "Färbemittel" bezeichnet. Diese Begriffe sind dabei nicht als beschränkend zu verstehen, so daß ein "Blondiermittel" neben der entfärbenden Wirkung durchaus auch zusätzlich färbende Eigenschaften aufweisen kann, während ein "Färbemittel" auch aufhellende Eigenschaften aufweisen kann.

Erfindungsgemäße Mittel enthalten ein Alkalisierungsmittel, welches mit einer Hülle umgeben ist.

Das Alkalisierungsmittel dient zur Einstellung eines alkalischen pH-Wertes der Anwendungsmischung. Erfindungsgemäß können die dem Fachmann die für Blondier- und/oder Färbemittel bekannten, üblichen Alkalisierungsmittel wie Ammonium-, Alkalimetall- und Erdalkalimetalhydroxyde, -carbonate, -hydrogencarbonate, -hydroxycarbonate, -silikate, insbesondere -metasilikate, sowie Alkaliphosphate verwendet werden. Auch kurzkettige Amine wie Monoethanolamine, 3-Amino-2-methylpropanol oder alkalisch reagierende Aminosäuren wie Arginin, Ornithin und Lysin sind einsetzbar. In einer bevorzugten Ausführungsform enthalten die erfindungsgemäßen Mittel mindestens zwei unterschiedliche Alkalisierungsmittel. Dabei können Mischungen beispielsweise aus einem Metasilikat und einem Hydroxycarbonat bevorzugt sein.

Die erfindungsgemäßen Mittel enthalten Alkalisierungsmittel (berechnet als unbeschichtetes Alkalisierungsmittel) bevorzugt in Mengen von 1-30 Gew.-%, insbesondere 2-25 Gew.-%, jeweils bezogen auf das gesamte Mittel.

Das beschichtete Alkalisierungsmittel, das in den erfindungsgemäßen Mitteln enthalten ist, umfaßt einen Partikelkern, der ein oder mehrere Alkalisierungsmittel enthält und eine diesen Kern umgebende Hülle, wobei die Hülle zu mindestens 50 Gew.-%, vorzugsweise zu mindestens 70 Gew.-%, weiter bevorzugt zu mindestens 90 Gew.-% und insbesondere zu 100 Gew.-% ihres Gewichts aus Fettsäuren besteht. Diese Stoffgruppen sowie besonders bevorzugte Vertreter werden nachstehend beschrieben.

Der Begriff "Fettsäuren" ist im Rahmen der vorliegenden Erfindung die Gruppenbezeichnung für aliphatische, gesättigte oder ungesättigte Carbonsäuren mit nahezu ausschließlich unverzweigter Kohlenstoff-Kette, Erfindungsgemäß vorzugsweise einsetzbar sind insbesondere die bei Raumtemperatur (20°C) festen Vertreter oder Mischungen von Fettsäuren, die bei 20°C fest sind. Vorzugsweise weisen die erfindungsgemäß eingesetzten Fettsäuren oder Fettsäuregemische höhere Schmelzpunkte auf, beispielsweise oberhalb von 30°C oder bevorzugt oberhalb von 40°C.

Erfindungsgemäß bevorzugte Mittel sind dadurch gekennzeichnet, daß die Hülle als Fettsäuren Stearinsäure und/oder Palmitinsäure und/oder Ölsäure und/oder Isostearinsäure oder Gemische dieser Säuren enthält.

Unabhängig von der Art der eingesetzten Beschichtungsmaterialien sind erfindungsgemäße Mittel bevorzugt, bei denen die Hülle aus Stoffen oder Stoffgemischen besteht, die einen Schmelzpunkt oder Schmelzbereich zwischen 30 und 100°C, vorzugsweise zwischen 40 und 99°C und insbesondere zwischen 50 und 95°C aufweisen.

Die erfindungsgemäßen Blondier- bzw. Aufhell- und Färbemittel enthalten vorzugsweise zusätzlich eine Peroxoverbindung. Die Auswahl dieser Peroxoverbindung unterliegt prinzipiell keinen Beschränkungen. Bevorzugte Peroxoverbindungen sind Wasserstoffperoxid (H₂O₂), beispielsweise in Form einer wäßrigen Lösung oder in Form eines H₂O₂-Adduktes an feste Träger, wobei Harnstoffperhydrat oder Natriumcarbonat-Peroxohydrat ("Natriumpercarbonat") eine besondere Bedeutung besitzen. Neben Wasserstoffperoxid oder an seiner Stelle können auch andere Peroxoverbindungen in den erfindungsgemäßen Mitteln enthalten sein.

Übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten.

Bevorzugte erfindungsgemäße Mittel sind daher dadurch gekennzeichnet, daß sie zusätzlich eine feste Peroxoverbindung enthalten, die vorzugsweise ausgewählt ist aus Wasserstoffperoxid-Anlagerungsverbindungen an feste Träger, Ammonium- und Alkalimetall-persulfaten und - peroxidisulfaten, wobei besonders bevorzugte erfindungsgemäße Mittel mindestens 2 verschiedene Peroxidisulfate enthalten.

Die Peroxoverbindungen sind in den erfindungsgemäßen Blondier- bzw. Aufhell- und Färbemitteln bevorzugt in Mengen von 2-80 Gew.-%, insbesondere in Mengen von 5-50 Gew.-% enthalten.

Weiterhin hat es sich als vorteilhaft erwiesen, wenn die erfindungsgemäßen Blondiermittel nichtionogene grenzflächenaktive Stoffe enthalten. Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen.

Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, daß die gewählten Substanzen bevorzugt frei von Lösungsmitteln, Stellmitteln und anderen Begleitstoffen sein sollen.

Bevorzugte nichtionogene grenzflächenaktive Stoffe sind
- alkoxylierte Fettalkohole mit 8 bis 22, insbesondere 10 bis 16, Kohlenstoffatomen in der Fettalkylgruppe und 1 bis 30, insbesondere 1 bis 15, Ethylenoxid- und/oder Propylenoxid-Einheiten. Bevorzugte Fettalkylgruppen sind beispielsweise Lauryl-, Myristyl-, Cetyl-, aber auch Stearyl-, Isostearyl- und Oleylgruppen. Besonders bevorzugte Verbindungen dieser Klasse sind beispielsweise Laurylalkohol mit 2 bis 4 Ethylenoxid-Einheiten, Oleyl- und Cetylalkohol mit jeweils 5 bis 10 Ethylenoxideinheiten, Cetyl- und Stearylalkohol sowie deren Mischungen mit 10 bis 30 Ethylenoxideinheiten sowie das Handelsprodukt Aethoxal^{®}B (Henkel), ein Laurylalkohol mit jeweils 5 Ethylenoxid- und Propylenoxideinheiten. Neben den üblichen alkoxylierten Fettalkoholen können auch sogenannte "endgruppenverschlossene" Verbindungen erfindungsgemäß eingesetzt werden. Bei diesen Verbindungen weist die Alkoxygruppe am Ende keine OH-Gruppe auf, sondern ist in Form eines Ethers, insbesondere eines C1-C4-Alkyl-Ethers, "verschlossen". Ein Beispiel für eine solche Verbindung ist das Handelsprodukt Dehypon^{®}LT 054, ein C₁₂₋₁₈-Fettalkoholol + 4,5 Ethylenoxid-butylether.
- alkoxylierte Fettsäuren mit 8 bis 22, insbesondere 10 bis 16, Kohlenstoffatomen in der Fettsäuregruppe und 1 bis 30, insbesondere 1 bis 15, Ethylenoxid- und/oder Propylenoxid-Einheiten. Bevorzugte Fettsäuren sind beispielsweise Laurin-, Myristin-, Palmitin-, Stearin-, Isostearin- und Ölsäure.
- alkoxylierte, bevorzugt propoxylierte und insbesondere ethoxylierte, Mono-, Di- und Triglyceride. Beispiele für bevorzugte Verbindungen sind Glycerinmonolaurat + 20 Ethylenoxid und Glycerinmonostearat + 20 Ethylenoxid.
- Polyglycerinester und alkoxylierte Polyglycerinester. Bevorzugte Verbindungen dieser Klasse sind beispielsweise Poly(3)glycerindiisostearat (Handelsprodukt: Lameform^{®}TGI (Henkel)) und Poly(2)glycerinpolyhydroxystearat (Handelsprodukt: Dehymuls^{®}PGPH (Henkel)).
- Sorbitan-Fettsäureester und alkoxylierte Sorbitan- Fettsäureester wie beispielsweise Sorbitanmonolaurat und Sorbitanmonolaurat + 20 Ethylenoxid (EO).
- Alkylphenole und Alkylphenolalkoxylate mit 6 bis 21, insbesondere 6 bis 15, Kohlenstoffatomen in der Alkylkette und 0 bis 30 Ethylenoxid- und/oder Propylenoxid-Einheiten. Bevorzugte Vertreter dieser Klasse sind beispielsweise Nonylphenol + 4 EO, Nonylphenol + 9 EO, Octylphenol + 3 EO und Octylphenol + 8 EO.

Besonders bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.

Als besonders vorteilhaft haben sich erfindungsgemäße Mittel erwiesen, die nichtionogene grenzflächenaktive Substanzen in Mengen von 1 - 5 Gew.-% enthalten.

Weiterhin können die erfindungsgemäßen Blondiermittel alle in solchen Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, kationischen oder nichtionischen Tensiden auszuwählen. Anionische Tenside können dabei ganz besonders bevorzugt sein.

Bevorzugte anionische Tenside sind Alkylsulfate, Ethercarbonsäuresalze mit 10 bis 18 C-Atomen in der Alkylgruppe und bis zu 12 Glykolethergruppen im Molekül wie C₁₂H₂₅-(C₂H₄O)₆-CH₂-COONa sowie insbesondere Salze von gesättigten und speziell ungesättigten C8-C22-Carbonsäuren wie Ölsäure, Stearinsäure, Isostearinsäure und Palmitinsäure.

Diese anionischen Tenside sollten bevorzugt in fester, insbesondere Pulverform vorliegen. Ganz besonders bevorzugt sind dabei bei Raumtemperatur feste Seifen, insbesondere Natriumstearat. Diese liegen bevorzugt in Mengen von 5 bis 20 Gew.-%, insbesondere 10 bis 15 Gew.-.%, vor.

Als nichtionische Tenside eignen sich insbesondere C8-C22-Alkylmono- und oligoglycoside und deren ethoxylierte Analoga. Insbesondere die nichtethoxylierten Verbindungen haben sich als besonders geeignet erwiesen.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie das unter der Bezeichnung Dehyquart^{®}F 75 in Abmischung mit Cetearylalkohle erhältliche Distearoylethylhydroxyethylammoniummethosulfat.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether und andere, als Feststoff stabile bzw. im Handel erhältliche Verbindungen,
- zwitterionische und amphotere Polymere, die als Feststoffe stabil bzw. bevorzugt als Handelsprodukte erhältlich sind,
- anionische Polymere wie beispielsweise Polyacrylsäuren, vernetzte Polyacrylsäuren und Vinylacetat/Crotonsäure-Copolymere, sofern diese als Feststoffe stabil bzw. bevorzugt im Handel erhältlich sind,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle
- Proteinhydrolysate, insbesondere Elastin-, Kollagen-, Keratin-, Milcheiweiß-, Sojaproteinund Weizenproteinhydrolysate, deren Kondensationsprodukte mit Fettsäuren sowie quaternisierte Proteinhydrolysate,
- Parfümöle, Dimethylisosorbid und Cyclodextrine,
- Farbstoffe zum Einfärben der Zubereitungen,
- Wirkstoffe wie Panthenol, Pantothensäure, Allantoin, Pyrrolidoncarbonsäuren und deren Salze,
- Cholesterin,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Carbonate, Hydrogencarbonate, Guanidine, Harnstoffe sowie primäre, sekundäre und tertiäre Phosphate,

Die Auswahl dieser weiteren Stoffe wird der Fachmann gemäß der gewünschten Eigenschaften der Mittel treffen.

Die erfindungsgemäßen Mittel können zusätzlich ein oder mehrere Farbstoffvorprodukte enthalten.

Erfindungsgemäße Mittel, die zusätzlich mindestens ein Oxidationsfarbstoffvorprodukt vom Entwicklertyp und/gegebenenfalls mindestens ein Oxidationsfarbstoffvorprodukt vom Kupplertyp enthalten, sind dabei bevorzugt.

Hinsichtlich der in den erfindungsgemäßen Blondiermitteln einsetzbaren weiteren Farbstoffvorprodukte unterliegt die vorliegende Erfindung keinerlei Einschränkungen. Die erfindungsgemäßen Blondiermittel können als weitere Farbstoffvorprodukte
- Oxidationsfarbstoffvorprodukte vom Entwickler- und/oder Kuppler-Typ, und
- Vorstufen naturanaloger Farbstoffe, wie Indol- und Indolin-Derivate,
sowie Mischungen von Vertretern dieser Gruppen enthalten.

In einer ersten bevorzugten Ausführungsform enthält das Blondiermittel weiterhin mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 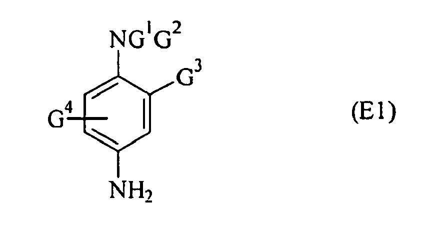 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁-bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁-bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁-bis C₄-Hydroxyalkylgruppe eine Hydroxymethyl-, eine 2-Hydroxyethyl-, eine 3-Hydroxypropyl-oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfingdungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaitige Gruppen der Formel (E1) sind insbesondere die Aminogruppen, C₁- bis C₄-Monoalkylaminogruppen, C₁-bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Mitteln gemäß der Erfindung verwendet werden können, kann man insbesondere die Verbindungen nennen, die der folgenden Formel (E2) entsprechen, sowie ihre physiologisch verträglichen Salze: 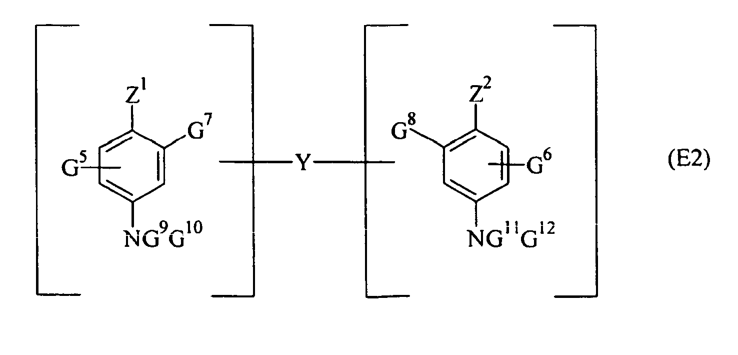 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁-bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 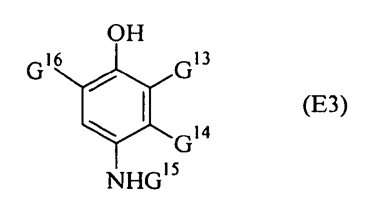 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁-bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(□-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-PyrimidinDerivaten und ihren physiologisch verträglichen Salzen.

Bevorzugte Pyridin-Derivate sind insbesondere die Verbindungen 2,5-Diamino-pyridin, 2-(4'-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(□-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 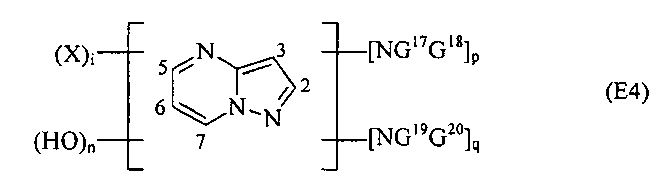 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)aminoalkylrest, einen Aminorest, einen C₁- bis C₄-Alkyl- oder Di-(C₁- bis C₄-hydroxyalkyl)aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

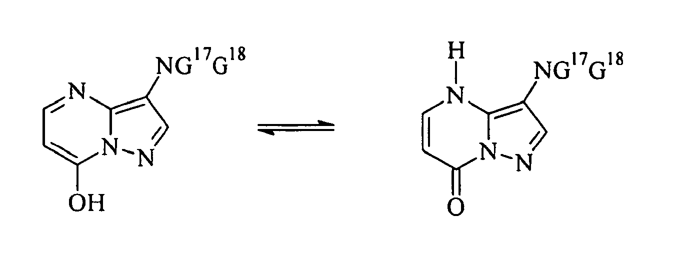

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)amino]-ethanol;
- 2-[(7-Aminopyrazol-[1,5-a]-pyrimidin-3-yl)-(2-hydroxy-ethyl)amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomeres Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

In einer weiteren bevorzugten Ausführungsform enthalten die erfindungsgemäßen Mittel mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Mittel mindestens ein Indol- und/oder Indolinderivat.

Besonders gut als Vorstufen naturanaloger Haarfarbstoffe geeignet sind Derivate des 5,6-Dihydroxyindolins der Formel (VIIIa), 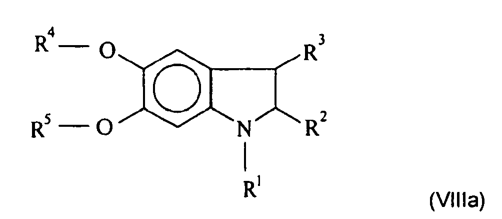 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (VIIIb), 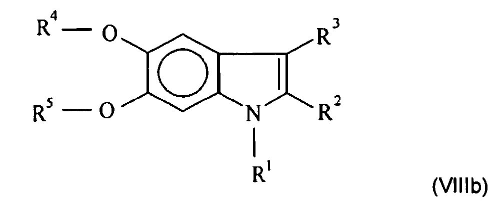 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Mitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Haarfärbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Bevorzugte erfindungsgemäße Mittel sind dadurch gekennzeichnet, daß sie mindestens einen Farbstoffvorläufer aus den Gruppen der aromatischen und heteroaromatischen Diamine, Aminophenole, Naphthole, Polyphenole CH-aciden Kupplerkomponenten und ihrer Derivate in Mengen von 0,01 bis 25 Gew.%, vorzugsweise von 0,5 bis 10 Gew.%, insbesondere von 1 bis 5 Gew.-%, jeweils bezogen auf das gesamte Mittel, enthalten.

Neben dem/den erfindungsgemäß enthaltenen beschichteten Alkalisierungsmittel(n) können die erfindungsgemäßen Mittel zur Nuancierung einen oder mehrere direktziehende Farbstoffe enthalten. Direktziehende Farbstoffe sind üblicherweise Nitrophenylendiamine, Nitroaminophenole, Azofarbstoffe. Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.
Entsprechende erfindungsgemäße Mittel, die dadurch gekennzeichnet sind, daß sie mindestens einen direktziehenden Farbstoff aus der Gruppe der kationischen (basischen) Farbstoffe, vorzugsweise Basic Blue 6, C.I.-No. 51,175; Basic Blue 7, C.I.-No. 42,595; Basic Blue 9, C.I.-No. 52,015; Basic Blue 26, C.I.-No. 44,045; Basic Blue 41, C.I.-No. 11,154; Basic Blue 99, C.I-No. 56,059; Basic Brown 4, C.I.-No. 21,010; Basic Brown 16, C.I.-No. 12,250; Basic Brown 17, C.L-No. 12,251; Basic Green 1, C.I.-No. 42,040; Basic Orange 31; Basic Red 2, C.I.-No. 50,240; Basic Red 22, C.I.-No. 11,055; Basic Red 46; Basic Red 51; Basic Red 76, C.I.-No. 12,245; Basic Violet 1, C.I.-No. 42,535; Basic Violet 2; Basic Violet 3, C.I.-No. 42,555; Basic Violet 10, C.I.-No. 45,170; Basic Violet 14, C.I.-No. 42,510; Basic Yellow 57, C.I.-No. 12,719; Basic Yellow 87 und/oder der anionischen (sauren) Farbstoffe, und/oder der nichtionischen Farbstoffe, vorzugsweise Acid Black 1, C.I.-No. 20,470; Acid Black 52; Acid Blue 7; Acid Blue 9, C.I.-No. 42,090; Acid Blue 74, C.I.-No. 73,015, Acid Red 18, C.I.-No. 16,255; Acid Red 23; Acid Red 27, C.I.-No. 16,185; Acid Red 33; Acid Red 52; Acid Red 87, C.I.-No. 45,380; Acid Red 92, C.I.-No. 45,410; Acid Orange 3; Acid Orange 7; Acid Violet 43, C.I.-No. 60,730; Acid Yellow 1, C.I.-No. 10,316; Acid Yellow 10; Acid Yellow 23, C.I.-No. 19,140; Acid Yellow 3, C.I.-No. 47,005; Acid Yellow 36; D& C Brown No. 1, C.I.-No. 20,170 (Acid Orange 24); D&C Green No. 5, C.I.-No. 61,570 (Acid Green G); D&C Orange No. 4, C.I.-No. 15,510 (Acid Orange II); D&C Orange No. 10, C. I.-No. 45,425 : 1 (Solvent Red 73); D&C Orange No. 11, C.I.-No. 45,425 (Acid Red 95); D&C Red No. 21, C.I.-No. 45,380 : 2 (Solvent Red 43); D&C Red No. 27, C.I.-No. 45,410 : 1 (Solvent Red 48); D&C Red No. 33, C.I.-No. 17,200 (Acid Red 2A, Acid Red B); D&C Yellow No. 7, C. I.-No. 45,350 : 1 (Solvent Yellow 94); D&C Yellow No. 8, C.I.-No. 45,350 (Acid Yellow 73); FD& C Red No. 4, C.I.-No. 14,700 (Food Red 4); FD&C Yellow No. 6, C.I.-No. 15,985 (Food Yellow 3); Food Green 3; Pigment Red 57-1; Disperse Black 9; Disperse Blue 1; Disperse Blue 3; Disperse Violet 1; Disperse Violet 4; HC Orange 1; HC Red 1; HC Red 3; HC Red 13; HC Yellow 2; HC Yellow 4; Na-Pikramat; 1,4-Bis-(2'-hydroxyethyl)amino- 2-nitro-p-phenylendiamin; HC Yellow 5; HC Blue 2; HC Blue 12; 4-Amino-3-nitrophenol; HC Yellow 6; HC Yellow 12; 2-Nitro-1-(2'hydroxyethyl)amino-4-methylbenzol; 2-Nitro-4-amino-diphenylamin-2-carbonsäure; 2-Amino-6-chlor-4-nitrophenol; HC Red BN; 6-Nitro-1,2,3,4-tetranitrochinoxalin; o-Nitro-p-phenylendiamin; p-Nitro-m-phenylendiamin; HC Red B 54; HC Red 10; HC Red 11; HC Red 13; 2-(2'-Hydroxyethyl)amino-1-hydroxy-4,6-dinitrobenzol; 4-Ethylamino-3-nitrobenzoesäure; 2-Chlor-6-ethylamino-4-nitrophenol; 2-Hydroxy-1,4-napthochinon; 1-Propen-(4-amino-2-nitrophenyl)amin; Isatin: N-methylylisatin; HC Violet 1; HC Violet 2; 4-Nitrophenyl-aminoethylharnstoff in Mengen von 0,01 bis 25 Gew.%, vorzugsweise von 0,5 bis 10 Gew.%, insbesondere von 1 bis 5 Gew.-%, jeweils bezogen auf das gesamte Mittel, enthalten, sind bevorzugte Ausführungsformen der vorliegenden Erfindung.

Unter den vorstehend genannten Farbstoffen sind einige Vertreter besonders bevorzugt, weshalb weiter bevorzugte erfindungsgemäße Mittel, die dadurch gekennzeichnet sind, daß sie mindestens einen Direktzieher, ausgewählt aus Basic Blue 7, Basic Blue 99, Basic Violet 14, Basic Brown 16, Basic Brown 17, Basic Orange 31, Basic Red 46, Basic Red 51, Basic Red 76, Basic Yellow 57, Basic Yellow 87, Acid Black 1, Acid Blue 7, Acid Violet 43, Acid Red 23, Acid Red 52, Acid Orange 7, Acid Yellow 1, Acid Yellow 10, Acid Yellow 36, Food Green 3, Pigment Red 57-1, Disperse Black 9, Disperse Blue 1, Disperse Blue 3, Disperse Violet 1, Disperse Violet 4, HC Orange 1, HC Red 1, HC Red 3, HC Red 13, HC Yellow 2, HC Yellow 4, Na-Pikramat, 1,4-Bis-(2'-hydroxyethyl)amino-2-nitro- p-phenylendiamin, HC Yellow 5, HC Blue 2, HC Blue 12, 4-Amino-3-nitrophenol, HC Yellow 6, HC Yellow 12, 2-Nitro-1-(2'hydroxyethyl)amino-4-methylbenzol, 2-Nitro-4-amino-diphenylamin-2-carbonsäure, 2-Amino-6-chlor-4-nitrophenol, HC Red BN; 6-Nitro-1,2,3,4-tetranitrochinoxalin, o-Nitro-p-phenylendiamin, p-Nitro-m-phenylendiamin, HC Red B 54, HC Red 10, HC Red 11, HC Red 13, 2-(2'-Hydroxyethyl)amino-1-hydroxy-4,6-dinitrobenzol, 4-Ethylamino-3-nitrobenzoesäure, 2-Chlor-6-ethylamino-4-nitrophenol, 2-Hydroxy-1,4-napthochinon, 1-Propen-(4-amino-2-nitrophenyl)amin, Isatin, N-Methylylisatin, HC Violet 1, HC Violet 2, 4-Nitrophenyl-aminoethylharnstoff in Mengen von 0,01 bis 25 Gew.%, vorzugsweise von 0,5 bis 10 Gew.%, insbesondere von 1 bis 5 Gew.-%, jeweils bezogen auf das gesamte Mittel, enthalten, bevorzugt sind.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

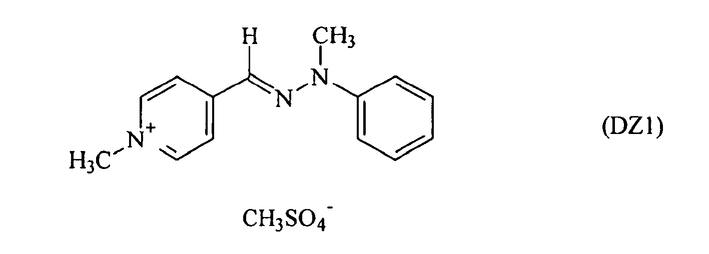

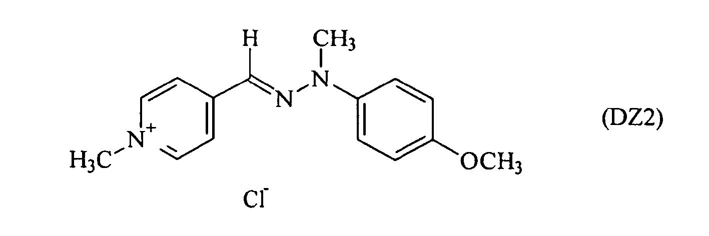

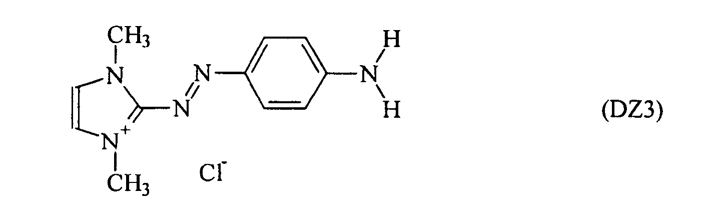

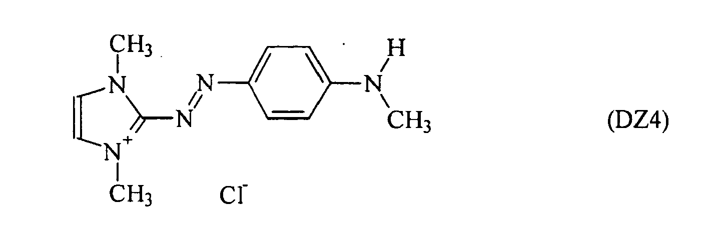

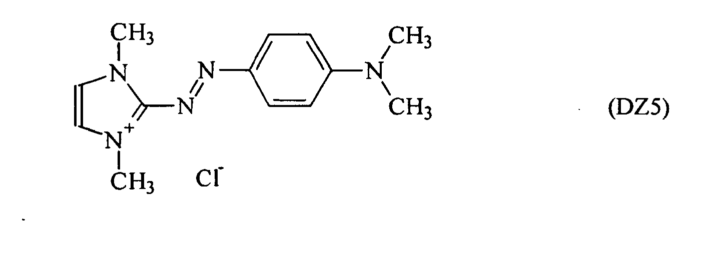

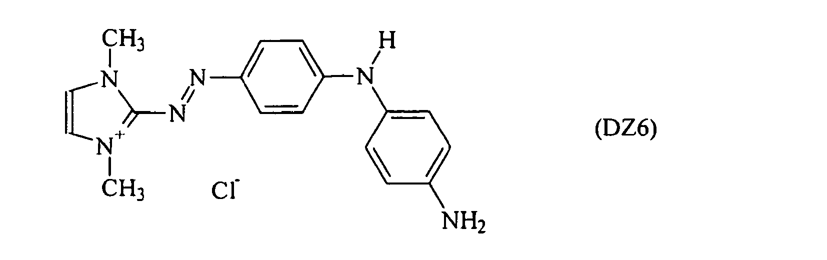

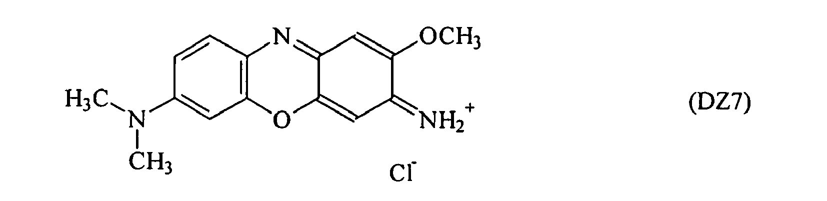

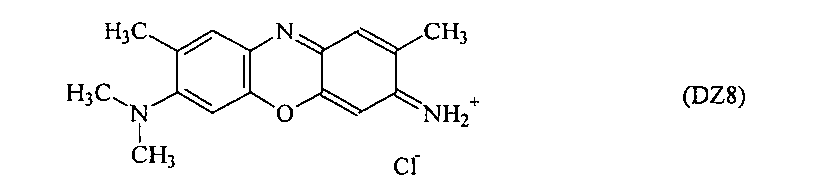

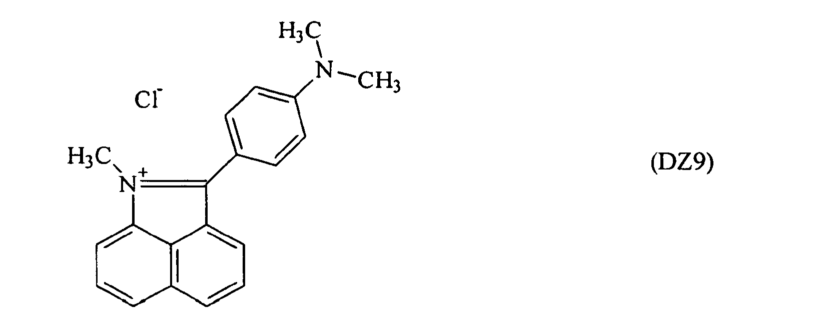

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Mittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Mitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

In einer weiteren Ausführungsform enthält das erfindungsgemäße Mittel zusätzlich eine Kombination aus Komponente
A Verbindungen, die eine reaktive Carbonylgruppe enthalten mit Komponente
B Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden.

Erfindungsgemäße Verbindungen mit einer reaktiven Carbonylgruppe (im Folgenden auch reaktive Carbonylverbindungen oder Komponente A genannt) besitzen mindestens eine Carbonylgruppe als reaktive Gruppe, welche mit den Verbindungen der Komponente B unter Ausbildung einer beide Komponenten verknüpfenden chemischen Bindung reagiert. Ferner sind erfindungsgemäß auch solche Verbindungen als Komponente A umfaßt, in denen die reaktive Carbonylgruppe derart derivatisiert bzw. maskiert ist, daß die Reaktivität des Kohlenstoffatoms der derivatisierten bzw. maskierten Carbonylgruppe gegenüber der Komponente B stets vorhanden ist. Diese Derivate sind bevorzugt Kondensationsverbindungen von reaktiven Carbonylverbindungen mit
a) Aminen und deren Derivate unter Bildung von Iminen oder Oximen als Kondensationsverbindung
b) von Alkoholen unter Bildung von Acetalen oder Ketalen als Kondensationsverbindung.

Die Komponente A wird bevorzugt ausgewählt aus der Gruppe, die gebildet wird aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxy-acetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxybenzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron, Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxy-benzaldehyd, 4-Hydroxy-3,5-dimethyl-benzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxybenzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 2-Methoxy-zimtaldehyd, 4-Methoxy-zimtaldehyd, 4-Hydroxy-3-methoxy-zimtaldehyd, 3,5-Dimethoxy-4-hydroxy-zimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylaminozimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd, Piperonal, 2,3,6,7-Tetrahydro-1H,5H-benzo[ ij]chinolizin-9-carboxaldehyd, 2,3,6,7-Tetrahydro-8-hydroxy-1H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon, Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzy-liden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-meth-oxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)-hexa-3,5-dien-2-on, 6-(4-Pyrrolidinophenyl)hexa-3,5-dien-2-on, 5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formyl-benzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methyl-acridinium-, 4-(2'-Formylvinyl)-1-methylpyridinium-, 1,3-Dimethyl-2-(4'-formylphenyl)-benzimidazo-lium-, 1,3-Dimethyl-2-(4'-formylphenyl)-imidazolium-, 2-(4'-Formylphenyl)-3-methylben-zothiazolium-, 2-(4'-Acetylphenyl)-3-methylbenzothiazolium-, 2-(4'-Formylphenyl)-3-methyl-benzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-chlor-2-(4'-formylphenyl)-3-methylbenzothiazolium-, 2-(4'-Formylphenyl)-3,5-dimethylbenzothiazolium-benzolsulfonat, -p-toluolsulfonat, -methansulfonat, -perchlorat, -sulfat, -chlorid, -bromid, -iodid, - tetrachlorozinkat, -methylsulfat-, trifluormethansulfonat, -tetrafluoroborat, Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Als CH-acide werden im allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Unter CH-acide Verbindungen fallen erfindungsgemäß auch Enamine, die durch alkalische Behandlung von quaternierten N-Heterozyklen mit einer in Konjugation zum quartären Stickstoff stehenden CH-aciden Alkylgruppe entstehen.

Die CH-aciden Verbindungen der Komponente B sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

Die weiteren bevorzugten Verbindungen der Komponente B sind dem Fachmann aus der Druckschrift WO-A1-99/18916 (Seiten 6 bis 9) bekannt, auf die hier ausdrücklich und vollinhaltlich Bezug genommen wird.

Zusammenfassend sind erfindungsgemäße Mittel bevorzugt, die mindestens einen direktziehenden Farbstoff aus der Gruppe der kationischen (basischen) Farbstoffe, vorzugsweise Basic Blue 6, C.I.-No. 51,175; Basic Blue 7, C.I.-No. 42,595; Basic Blue 9, C.I.-No. 52,015; Basic Blue 26, C.I.-No. 44,045; Basic Blue 41, C.I.-No. 11,154; Basic Blue 99, C.I.-No. 56,059; Basic Brown 4, C.I.-No. 21,010; Basic Brown 16, C.I.-No. 12,250; Basic Brown 17, C.I.-No. 12,251; Basic Green 1, C.I.-No. 42,040; Basic Orange 31; Basic Red 2, C.I.-No. 50,240; Basic Red 22, C.I.-No. 11,055; Basic Red 46; Basic Red 51; Basic Red 76, C.I.-No. 12,245; Basic Violet 1, C.I.-No. 42,535; Basic Violet 2; Basic Violet 3, C.I.-No. 42,555; Basic Violet 10, C.I.-No. 45,170; Basic Violet 14, C.I.-No. 42,510; Basic Yellow 57, C.I.-No. 12,719; Basic Yellow 87 und/oder der anionischen (sauren) Farbstoffe, und/oder der nichtionischen Farbstoffe, vorzugsweise Acid Black 1, C.I.-No. 20,470; Acid Black 52; Acid Blue 7; Acid Blue 9, C.I.-No. 42,090; Acid Blue 74, C.I.-No. 73,015, Acid Red 18, C.I.-No. 16,255; Acid Red 23; Acid Red 27, C.I.-No. 16,185; Acid Red 33; Acid Red 52; Acid Red 87, C.I.-No. 45,380; Acid Red 92, C.I.-No. 45,410; Acid Orange 3; Acid Orange 7; Acid Violet 43, C.I.-No. 60,730; Acid Yellow 1, C.I.-No. 10,316; Acid Yellow 10; Acid Yellow 23, C.I.-No. 19,140; Acid Yellow 3, C.I.-No. 47,005; Acid Yellow 36; D& C Brown No. 1, C.I.-No. 20,170 (Acid Orange 24); D&C Green No. 5, C.I.-No. 61,570 (Acid Green G); D&C Orange No. 4, C.I.-No. 15,510 (Acid Orange II); D&C Orange No. 10, C. I.-No. 45,425 : 1 (Solvent Red 73); D&C Orange No. 11, C.I.-No. 45,425 (Acid Red 95); D&C Red No. 21, C.I.-No. 45,380 : 2 (Solvent Red 43); D&C Red No. 27, C.I.-No. 45,410 : 1 (Solvent Red 48); D&C Red No. 33, C.I.-No. 17,200 (Acid Red 2A, Acid Red B); D&C Yellow No. 7, C. I.-No. 45,350 : 1 (Solvent Yellow 94); D&C Yellow No. 8, C.I-No. 45,350 (Acid Yellow 73); FD& C Red No. 4, C.I.-No. 14,700 (Food Red 4); FD&C Yellow No. 6, C.I.-No. 15,985 (Food Yellow 3); Food Green 3; Pigment Red 57-1; Disperse Black 9; Disperse Blue 1; Disperse Blue 3; Disperse Violet 1; Disperse Violet 4; HC Orange 1; HC Red 1; HC Red 3; HC Red 13; HC Yellow 2; HC Yellow 4; Na-Pikramat; 1,4-Bis-(2'-hydroxyethyl)amino- 2-nitro-p-phenylendiamin; HC Yellow 5; HC Blue 2; HC Blue 12; 4-Amino-3-nitrophenol; HC Yellow 6; HC Yellow 12; 2-Nitro-1-(2'hydroxyethyl)amino-4-methylbenzol; 2-Nitro-4-amino-diphenylamin-2-carbonsäure; 2-Amino-6-chlor-4-nitrophenol; HC Red BN; 6-Nitro-1,2,3,4-tetranitrochinoxalin; o-Nitro-p-phenylendiamin; p-Nitro-m-phenylendiamin; HC Red B 54; HC Red 10; HC Red 11; HC Red 13; 2-(2'-Hydroxyethyl)amino-1-hydroxy-4,6-dinitrobenzol; 4-Ethylamino-3-nitrobenzoesäure; 2-Chlor-6-ethylamino-4-nitrophenol; 2-Hydroxy-1,4-napthochinon; 1-Propen-(4-amino-2-nitrophenyl)amin; Isatin; N-methylylisatin; HC Violet 1; HC Violet 2; 4-Nitrophenyl-aminoethylharnstoff in Mengen von 0,01 bis 25 Gew.%, vorzugsweise von 0,5 bis 10 Gew.%, insbesondere von 1 bis 5 Gew.-%, jeweils bezogen auf das gesamte Mittel, enthalten.

Weiter bevorzugte erfindungsgemäße Mittel sind dadurch gekennzeichnet, daß sie mindestens einen Direktzieher, ausgewählt aus Basic Blue 7, Basic Blue 99, Basic Violet 14, Basic Brown 16, Basic Brown 17, Basic Orange 31, Basic Red 46, Basic Red 51, Basic Red 76, Basic Yellow 57, Basic Yellow 87, Acid Black 1, Acid Blue 7, Acid Violet 43, Acid Red 23, Acid Red 52, Acid Orange 7, Acid Yellow 1, Acid Yellow 10, Acid Yellow 36, Food Green 3, Pigment Red 57-1, Disperse Black 9, Disperse Blue 1, Disperse Blue 3, Disperse Violet 1, Disperse Violet 4, HC Orange 1, HC Red 1, HC Red 3, HC Red 13, HC Yellow 2, HC Yellow 4, Na-Pikramat, 1,4-Bis-(2'-hydroxyethyl)amino-2-nitro- p-phenylendiamin, HC Yellow 5, HC Blue 2, HC Blue 12, 4-Amino-3-nitrophenol, HC Yellow 6, HC Yellow 12, 2-Nitro-1-(2'hydroxyethyl)amino-4-methylbenzol, 2-Nitro-4-amino-diphenylamin-2-carbonsäure, 2-Amino-6-chlor-4-nitrophenol, HC Red BN; 6-Nitro-1,2,3,4-tetranitrochinoxalin, o-Nitro-p-phenylendiamin, p-Nitro-m-phenylendiamin, HC Red B 54, HC Red 10, HC Red 11, HC Red 13, 2-(2'-Hydroxyethyl)amino-1-hydroxy-4,6-dinitrobenzol, 4-Ethylamino-3-nitrobenzoesäure, 2-Chlor-6-ethylamino-4-nitrophenol, 2-Hydroxy-1,4-napthochinon, 1-Propen-(4-amino-2-nitrophenyl)amin, Isatin, N-Methylylisatin, HC Violet 1, HC Violet 2, 4-Nitrophenyl-aminoethylharnstoff in Mengen von 0,01 bis 25 Gew.%, vorzugsweise von 0,5 bis 10 Gew.%, insbesondere von 1 bis 5 Gew.-%, jeweils bezogen auf das gesamte Mittel, enthalten.

Auch erfindungsgemäße Mittel, die mindestens einen Farbstoffvorläufer aus den Gruppen der aromatischen und heteroaromatischen Diamine, Aminophenole, Naphthole, Polyphenole CH-aciden Kupplerkomponenten und ihrer Derivate in Mengen von 0,01 bis 25 Gew.%, vorzugsweise von 0,5 bis 10 Gew.%, insbesondere von 1 bis 5 Gew.-%, jeweils bezogen auf das gesamte Mittel, enthalten, sind erfindungsgemäß bevorzugt.

Zusätzlich können die erfindungsgemäßen Mittel weitere Inhaltsstoffe enthalten. Ein Einsatz bestimmter Metallionen oder -komplexe kann beispielsweise bevorzugt sein, um intensive Färbungen zu erhalten. Hier sind erfindungsgemäße Mittel bevorzugt, die zusätzlich Cu-, Fe-, Mn-Ru-Ionen oder Komplexe dieser Ionen enthalten.

Bevorzugte erfindungsgemäße Haarfärbe- und - aufhellungsmittel enthalten 0,0001 bis 2,5 Gew.-%, vorzugsweise 0,001 bis 1 Gew.-%, bezogen auf die Gesamtzusammensetzung des Mittels, mindestens einer Verbindung aus der Gruppe Kupferchlorid (CuCl₂), Kupfersulfat (CuSO₄), Eisen(II)sulfat, Mangan(II)sulfat, Mangan(II)chlorid, Kobalt(II)chlorid, Cersulfat, Cerchlorid, Vanadiumsulfat, Kaliumjodid, Natriumjodid, Lithiumchlorid, Kaliumdichromat, Magnesiumacetat, Calciumchlorid, Calciumnitrat, Bariumnitrat, Mangandioxid (MnO₂) und/oder Hydrochinon.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Ferner können die erfindungsgemäßen Blondiermittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Die Gegenwart von Oxidationsfarbstoffvorprdukten ist demnach keine zwingende Voraussetzung für einen Einsatz von Oxidationsmitteln in den erfindungsgemäßen Mitteln. Als Oxidationsmittel kommen Persulfate, Chlorite und insbesondere Wasserstoffperoxid oder dessen Anlagerungsprodukte an Harnstoff, Melamin sowie Natriumborat in Frage.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, lodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Bei einer Anwendung von Oxidationsmitteln wird das eigentliche Färbemittel zweckmäßigerweise unmittelbar vor der Anwendung durch Mischung der Zubereitung des Oxidationsmittels mit der Zubereitung, enthaltend die Verbindungen der Formel I und gegebenenfalls Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann ein erfindungsgemäßes Mittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein weiterer Gegenstand der vorliegenden Erfindung ist ein Verfahren zur Herstellung von Mitteln zum Aufhellen und/oder Färben von Keratinfasern umfassend die Schritte
a) Beschichten mindestens eines Alkalisierungsmittels mit einem Beschichtungsmittel unter Ausbildung einer Hülle, die zu mindestens 50 Gew.-%, vorzugsweise zu mindestens 70 Gew.-%, weiter bevorzugt zu mindestens 90 Gew.-% und insbesondere zu 100 Gew.-% ihres Gewichts aus Fettsäuren ausgewählt aus Caprylsäure, Pelargonsäure, Caprinsäure, Undecansäure, Laurinsäure, Tridecansaure, Tetradecansäure, Pentadecansäure, Palmitinsäure, Margarinsäure, Stearinsäure, Isostearinsäure, Nonadecansäure, Arachinsäure, Behensäure, Lignocerinsäure, Cerotinsäure, Melissinsäure, Crotonsäure, Ölsäure, Erucasäure, Sorbinsäure, Elaeostearinsäure, Docosahexaensäure besteht.
b) Abmischen des/der beschichteten Alkalisierungsmittel(s) mit weiteren Inhaltstoffen von Blondier- und/Färbemitteln für keratinische Fasern.

Bezüglich bevorzugter Ausführungsformen des erfindungsgemäßen Verfahrens gilt hinsichtlich der Stoffauswahl mutatis mutandis das zu den erfindungsgemäßen Mitteln Gesagte.

In weiter bevorzugten erfindungsgemäßen Verfahren enthält das beschichtete Alkalisierungsmittel (inklusive Beschichtung), bezogen auf sein Gewicht, 5 bis 80 Gew.-%, vorzugsweise 10 bis 75 Gew.-% und insbesondere 20 bis 70 Gew.-% Alkalisierungsmittel.

Im ersten Schritt des erfindungsgemäßen Verfahrens wird mindestens ein Alkalisierungsmittel mit mindestens einem der vorstehend genannten Stoffe beschichtet. Dieser Schritt läßt sich in den unterschiedlichsten Apparaturen problemlos durchführen. In einer geeigneten Misch- und Granuliervorrichtung, beispielsweise in entsprechenden Anlagen vom Typ eines Eirich-Mischers, eines Lödige-Mischers, beispielsweise eines Pflugscharmischers der Firma Lödige, oder eines Mischers der Firma Schugi, kann bei Umfangsgeschwindigkeiten der Mischorgane vorzugsweise zwischen 2 und 7 m/s (Pflugscharmischer) beziehungsweise 3 bis 50 m/s (Eirich, Schugi), insbesondere zwischen 5 und 20 m/s ein Feststoffbett vorgelegt und nachfolgend unter Zusatz des Beschichtungsmittels abgepudert werden. Dabei kann gleichzeitig in an sich bekannter Weise eine vorbestimmte Korngröße des Granulats eingestellt werden. Der Abpuderungsprozeß benötigt nur einen sehr kurzen Zeitraum von beispielsweise etwa 0,5 bis 10 Minuten, insbesondere etwa 0,5 bis 5 Minuten (Eirich-Mischer, Lödige-Mischer) zur Homogenisierung des Gemisches unter Ausbildung des rieselfähigen Granulates. Im Schugi-Mischer hingegen reicht normalerweise eine Verweilzeit von 0,5 bis 10 Sekunden aus, um ein rieselfähiges Granulat zu erhalten. Für die Durchführung dieses Verfahrensschritts geeignete Mischer sind beispielsweise Eirich^{®}-Mischer der Serien R oder RV (Warenzeichen der Maschinenfabrik Gustav Eirich, Hardheim), der Schugi^{®} Flexomix, die Fukae^{®} FS-G-Mischer (Warenzeichen der Fukae Powtech, Kogyo Co., Japan), die Lödige^{®} FM-, KM- und CB-Mischer (Warenzeichen der Lödige Maschinenbau GmbH, Paderborn) oder die Drais^{®}-Serien T oder K-T (Warenzeichen der Drais-Werke GmbH, Mannheim).

Es ist erfindungsgemäß auch möglich, mehrere der oben genannten Mischer in Reihe zu schalten. Insbesondere bieten sich hier folgende Kombinationen aufeinanderfolgender Mischer an:
- Lödige CB / Lödige KM
- Lödige KM / Schugi Flexomix
- Schugi Flexomix / Lödige KM / Schugi Flexomix
- Schugi Flexomix / Lödige CB
- Lödige CB / Lödige KM / Schugi Flexomix

Bevorzugte erfindungsgemäße Verfahren sind dadurch gekennzeichnet, daß Schritt a) in einem Mischergranulator durchgeführt wird, wobei das/die Alkalisierungsmittel in fester Form vorgelegt und einer Lösung, Dispersion und/oder Schmelze des/der Beschichtungsmaterialien beaufschlagt wird/werden.

In weiter bevorzugten erfindungsgemäßen Verfahren wird zusätzlich eine Granulierflüssigkeit aufgegeben, wobei die Granulierflüssigkeit vorzugsweise frei von Tensid(en) und Komplexbildner(n) ist.

Alternativ zum Einsatz eines Mischergranulators kann auch eine Wirbelschichtapparatur zur Ausbildung des Feststoffcoatings genutzt werden. Erfindungsgemäße Verfahren, bei denen die Beschichtung des/der Alkalisierungsmittel in einer Wirbelschichtapparatur durchgeführt wird, sind bevorzugt.

Auch hier kann gleichzeitig Flüssigkeit auf die Körner aufgebracht werden. Die Beschichtung kann dabei gleichzeitig mit der Trocknung erfolgen (beispielsweise in einer Wirbelschichtapparatur, in der die Granulate mit einer Lösung oder Dispersion mindestens eines der oben genannten Stoffe beaufschlagt und gleichzeitig getrocknet werden), es ist aber auch möglich und bevorzugt, die Trocknung nach der Beschichtung, also zeitlich anschließend an diese, durchzuführen.

Alternativ zum Partikelcoating (Abpuderung) kann auch eine dichtere Hülle erzeugt werden, indem eine Lösung oder Dispersion des/der oben genannten Stoffe(s) auf das/die Alkalisierungsmittel aufgebracht wird. Diese Lösung bzw. Dispersion kann auch weitere filmbildende Substanzen enthalten. Ein erfindungsgemäßes Verfahren, bei dem das/die Alkalisierungsmittel mit einer Lösung oder Dispersion von Fettsäuren ausgewählt aus Caprylsäure, Pelargonsäure, Caprinsäure, Undecansäure, Laurinsäure, Tridecansaure, Tetradecansäure, Pentadecansäure, Palmitinsäure, Margarinsäure, Stearinsäure, Isostearinsäure, Nonadecansäure, Arachinsäure, Behensäure, Lignocerinsäure, Cerotinsäure, Melissinsäure, Crotonsäure, Ölsäure, Erucasäure, Sorbinsäure, Elaeostearinsäure, Docosahexaensäure besprüht und anschließend getrocknet wird/, ist demnach eine weitere bevorzugte Ausführungsform der vorliegenden Erfindung.

Zur Ausbildung einer besonders dichten Umhüllung kann diese auch in zwei Schritten aufgebracht werden. Dabei ist es bevorzugt, daß die erste Hülle aus einem höher schmelzenden Material als die zweite Hülle besteht.. Bevorzugte Temperaturdifferenzen für die Schmelz- bzw. Erweichungsbereiche sind 5- 30 , besonders bevorzugt 7-20 und ganz besonders bevorzugt 10-15 Grad Celsius.

### Beispiele:

Folgende Rohstoffe wurden verwendet:
- Idranal^{®} III: Ethylendiamintetraessigsäure Dinatrium Salz·2 H₂O (INCI-Bezeichnung: Disodium EDTA) (Hersteller: Riedel De Haen)
- Britesil^{®} C20: Natriumsilikat (INCI-Bezeichnung: Sodium Silikate) (The PQ Corporation)

### 1.0 Herstellung des Kern-Hülle-Alkalisierungsmittels

26,6 g Britesil^{®} C 20 wurden bei einer Temperatur von 65°C mit 8 g einer Mischung aus Stearinsäure/Isostearinsäure überzogen. Das Gemisch wurde unter starkem Rühren auf Raumtemperatur abgekühlt.

Die Hülle des Alkalisierungsmittels enthielt 100 % Fettsäuren.

### 2.0 Wirksamkeitstest

Es wurden folgende Blondierpulverrezepturen nach bekanntem Verfahren hergestellt:

| | **E1** (erfindungsgemäß) | **V1** (Vergleich) |
|---|---|---|
| Ammoniumperoxidisulfat | 6,5 g | 6,5 g |
| Kaliumperoxidisulfat | 21,4 g | 21,4 g |
| Natriumperoxidisulfat | 7,8 g | 7,8 g |
| Kern-Hülle-Alkalisierungmittel | 17,3 g | - |
| Britesil^{®} C 20 | - | 13,3 g |
| Idranal^{®} III | 1,0 g | 1,0 g |

Zu 50 g einer Entwicklerdispersion, enthaltend 12 Gew.% Wasserstoffperoxid (Poly^{®} Blond Ultra der Fa. Schwarzkopf & Henkel), wurden 20 g eines der oben genannten Blondierpulver gegeben und die Mischung in der Applikationsflasche des Handelsprodukts 30 Sekunden lang geschüttelt. Nach einer Ruhezeit von 20 Sekunden wurde die Applikationsflasche geöffnet und abgestellt. Ab Öffnen der Flasche wurde die Zeit bis zum selbsttätigen Austritt des Flascheninhalts aus der Flasche gemessen.

Biondierpulver E1: Es wurde keine Gasentwicklung beobachtet. Nach 2 Stunden wurde der Versuch abgebrochen, ohne dass das Mittel aus der Flasche austrat.

Blondierpulver V1: Es wurde eine Gasentwicklung beobachtet. Nach 1 Stunde und 38 Minuten trat der Flascheninhalt selbsttätig aus.

## Patentansprüche

1. Mittel zum Aufhellen und/oder Färben von Keratinfasern, insbesondere menschlichen Haaren, enthaltend mindestens ein festes Alkalisierungsmittel, **dadurch gekennzeichnet, daß** das Alkalisierungsmittel einen Partikelkern umfaßt, der Alkalisierungsmittel enthält und eine diesen Kern umgebende Hülle aufweist, die zu mindestens 50 Gew.-%, vorzugsweise zu mindestens 70 Gew.-%, weiter bevorzugt zu mindestens 90 Gew.-% und insbesondere zu 100 Gew.-% ihres Gewichts aus Fettsäuren ausgewählt aus Caprylsäure, Pelargonsäure, Caprinsäure, Undecansäure, Laurinsäure, Tridecansäure, Tetradecansäure, Pentadecansäure, Palmitinsäure, Margarinsäure, Stearinsäure, Isostearinsäure, Nonadecansäure, Arachinsäure, Behensäure, Lignocerinsäure, Cerotinsäure, Melissinsäure, Crotonsäure, Ölsäure, Erucasäure, Sorbinsäure, Elaeostearinsäure, Docosahexaensäure,
besteht.

2. Mittel nach Anspruch 1, **dadurch gekennzeichnet, daß** die Hülle als Fettsäuren Stearinsäure und/oder Palmitinsäure und/oder Ölsäure und/oder Isostearinsäure oder Gemische dieser Säuren enthält.

3. Mittel nach einem der Ansprüche 1 oder 2, **dadurch gekennzeichnet, daß** die Hülle aus Stoffen oder Stoffgemischen besteht, die einen Schmelzpunkt oder Schmelzbereich zwischen 30 und 100°C, vorzugsweise zwischen 40 und 99°C und insbesondere zwischen 50 und 95°C aufweisen.

4. Mittel nach einem der Ansprüche 1 bis 3, **dadurch gekennzeichnet, daß** es zusätzlich eine feste Peroxoverbindung enthält, die vorzugsweise ausgewählt ist aus Wasserstoffperoxid-Anlagerungsverbindungen an feste Träger, Ammonium- und Alkalimetall-persulfaten und - peroxidisulfaten.

5. Mittel nach Anspruch 4, **dadurch gekennzeichnet, daß** es mindestens 2 verschiedene Peroxidisulfate enthält.

6. Mittel nach einem der Ansprüche 1 bis 5, **dadurch gekennzeichnet, daß** es mindestens einen direktziehenden Farbstoff aus der Gruppe der kationischen (basischen) Farbstoffe, vorzugsweise Basic Blue 6, C.I.-No. 51,175; Basic Blue 7, C,I.-No. 42,595; Basic Blue 9, C.I-No. 52,015; Basic Blue 26, C.I.-No. 44,045; Basic Blue 41, C.I.-No. 11,154; Basic Blue 99, C.I.-No. 56,059; Basic Brown 4, C.I.-No. 21,010; Basic Brown 16, C.I.-No. 12,250; Basic Brown 17, C.I-No. 12,251; Basic Green 1, C.I.-No. 42,040; Basic Orange 31; Basic Red 2, C.I.-No. 50,240; Basic Red 22, C.I,-No. 11,055; Basic Red 46; Basic Red 51; Basic Red 76, C.I.-No. 12,245; Basic Violet 1, C.I.-No. 42,535; Basic Violet 2; Basic Violet 3, C.I.-No. 42,555; Basic Violet 10, C.I.-No. 45,170; Basic Violet 14, C.I-No. 42,510; Basic Yellow 57, C.I.-No. 12,719; Basic Yellow 87 und/oder der anionischen (sauren) Farbstoffe, und/oder der nichtionischen Farbstoffe, vorzugsweise Acid Black 1, C.I-No. 20,470; Acid Black 52; Acid Blue 7; Acid Blue 9, C.I.-No. 42,090; Acid Blue 74, C.I.-No. 73,015, Acid Red 18, C.I.-No, 16,255; Acid Red 23; Acid Red 27, C.I.-No. 16,185; Acid Red 33; Acid Red 52; Acid Red 87, C.I.-No. 45,380; Acid Red 92, C.I.-No. 45,410; Acid Orange 3; Acid Orange 7; Acid Violet 43, C.I.-No. 60,730; Acid Yellow 1, C.I.-No. 10,316; Acid Yellow 10; Acid Yellow 23, C.I.-No. 19,140; Acid Yellow 3, C.I.-No. 47,005; Acid Yellow 36; D& C Brown No. 1, C.I.-No. 20,170 (Acid Orange 24); D&C Green No. 5, C.I.-No. 61,570 (Acid Green G); D&C Orange No. 4, C.I.-No. 15,510 (Acid Orange II); D&C Orange No. 10, C. I.-No. 45,425 : 1 (Solvent Red 73); D&C Orange No. 11, C.I.-No. 45,425 (Acid Red 95); D&C Red No. 21, C.I.-No. 45,380 ; 2 (Solvent Red 43); D&C Red No. 27, C.I.-No. 45,410 : 1 (Solvent Red 48); D&C Red No. 33, C.I.-No. 17,200 (Acid Red 2A, Acid Red B); D&C Yellow No. 7, C. I-No. 45,350 : 1 (Solvent Yellow 94); D&C Yellow No. 8, C.I.-No. 45,350 (Acid Yellow 73); FD& C Red No. 4, C.I.-No. 14,700 (Food Red 4); FD&C Yellow No. 6, C.I.-No. 15,985 (Food Yellow 3); Food Green 3; Pigment Red 57-1; Disperse Black 9; Disperse Blue 1; Disperse Blue 3; Disperse Violet 1; Disperse Violet 4; HC Orange 1; HC Red 1; HC Red 3; HC Red 13; HC Yellow 2; HC Yellow 4; Na-Pikramat; 1,4-Bis-(2'-hydroxyethyl)amino- 2-nitro-p-phenylendiamin; HC Yellow 5; HC Blue 2; HC Blue 12; 4-Amino-3-nitrophenol; HC Yellow 6; HC Yellow 12; 2-Nitro-1-(2'hydroxyethyl)amino-4-methylbenzol; 2-Nitro-4-amino-diphenylamin-2-carbonsäure; 2-Amino-6-chlor-4-nitrophenol; HC Red BN; 6-Nitro-1,2,3,4-tetranitrochinoxalin; o-Nitro-p-phenylendiamin; p-Nitro-m-phenylendiamin; HC Red B 54; HC Red 10; HC Red 11; HC Red 13; 2-(2'-Hydroxyethyl)amino-1-hydroxy-4,6-dinitrobenzol; 4-Ethylamino-3-nitrobenzoesäure; 2-Chlor-6-ethylamino-4-nitrophenol; 2-Hydroxy-1,4-napthochinon; 1-Propen-(4-amino-2-nitrophenyl)amin; Isatin; N-methylylisatin; HC Violet 1; HC Violet 2; 4-Nitrophenyl-aminoethylharnstoff in Mengen von 0,01 bis 25 Gew.%, vorzugsweise von 0,5 bis 10 Gew.%, insbesondere von 1 bis 5 Gew.-%, jeweils bezogen auf das gesamte Mittel, enthält.

7. Mittel nach einem der Ansprüche 1 bis 6, **dadurch gekennzeichnet, dass** es mindestens einen Direktzieher, ausgewählt aus Basic Blue 7, Basic Blue 99, Basic Violet 14, Basic Brown 16, Basic Brown 17, Basic Orange 31, Basic Red 46, Basic Red 51, Basic Red 76, Basic Yellow 57, Basic Yellow 87, Acid Black 1, Acid Blue 7, Acid Violet 43, Acid Red 23, Acid Red 52, Acid Orange 7, Acid Yellow 1, Acid Yellow 10, Acid Yellow 36, Food Green 3, Pigment Red 57-1, Disperse Black 9, Disperse Blue 1, Disperse Blue 3, Disperse Violet 1, Disperse Violet 4, HC Orange 1, HC Red 1, HC Red 3, HC Red 13, HC Yellow 2, HC Yellow 4, Na-Pikramat, 1,4-Bis-(2'-hydroxyethyl)amino-2-nitro- p-phenylendiamin, HC Yellow 5, HC Blue 2, HC Blue 12, 4-Amino-3-nitrophenol, HC Yellow 6, HC Yellow 12, 2-Nitro-1-(2'hydroxyethyl)amino-4-methylbenzol, 2-Nitro-4-amino-diphenylamin-2-carbonsäure, 2-Amino-6-chlor-4-nitrophenol, HC Red BN; 6-Nitro-1,2,3,4-tetranitrochinoxalin, o-Nitro-p-phenylendiamin, p-Nitro-m-phenylendiamin, HC Red B 54, HC Red 10, HC Red 11, HC Red 13, 2-(2'-Hydroxyethyl)amino-1-hydroxy-4,6-dinitrobenzol, 4-Ethylamino-3-nitrobenzoesäure, 2-Chlor-6-ethylamino-4-nitrophenol, 2-Hydroxy-1,4-napthochinon, 1-Propen-(4-amino-2-nitrophenyl)amin, Isatin, N-Methylylisatin, HC Violet 1, HC Violet 2, 4-Nitrophenyl-aminoethylharnstoff in Mengen von 0,01 bis 25 Gew.%, vorzugsweise von 0,5 bis 10 Gew.%, insbesondere von 1 bis 5 Gew.-%, jeweils bezogen auf das gesamte Mittel, enthält.

8. Mittel nach einem der Ansprüche 1 bis 7, **dadurch gekennzeichnet, daß** es mindestens einen Farbstoffvorläufer aus den Gruppen der aromatischen und heteroaromatischen Diamine, Aminophenole, Naphthole, Polyphenole CH-aciden Kupplerkomponenten und ihrer Derivate in Mengen von 0,01 bis 25 Gew.%, vorzugsweise von 0,5 bis 10 Gew.%, insbesondere von 1 bis 5 Gew.-%, jeweils bezogen auf das gesamte Mittel, enthält.

9. Verfahren zur Herstellung von Mitteln zum Aufhellen und/oder Färben von Keratinfasern, **gekennzeichnet durch** die Schritte
a) Beschichten mindestens eines Alkalisierungsmittels mit einem Beschichtungsmittel unter Ausbildung einer Hülle, die zu mindestens 50 Gew.-%, vorzugsweise zu mindestens 70 Gew.-%, weiter bevorzugt zu mindestens 90 Gew.-% und insbesondere zu 100 Gew.-% ihres Gewichts aus Fettsäuren ausgewählt aus Capryläure, Pelargonsäure, Caprinsäure, Undecansäure, Laurinsäure, Tridecansäure, Tetradecansäure, Pentadecansäure, Palmitinsäure, Margarinsäure, Stearinsäure, Isostearinsäure, Nonadecansäure, Arachinsäure, Behensäure, Lignocerinsäure, Cerotinsäure, Melissinsäure, Crotonsäure. Ölsäure, Erucasäure, Sorbinsäure, Elaeostearinsäure, Docosahexaensäure besteht,
b) Abmischen des/der beschichteten Alkalisierungsmittel(s) mit weiteren Inhaltstoffen von Blondier- und/Färbernitteln für keratinische Fasern.

10. Verfahren nach Anspruch 9, **dadurch gekennzeichnet, daß** das beschichtete Alkalisierungsmittel (inklusive Beschichtung), bezogen auf sein Gewicht, 5 bis 80 Gew.-%, vorzugsweise 10 bis 75 Gew.-% und insbesondere 20 bis 70 Gew.-% Alkalisierungsmittel enthält.

11. Verfahren nach einem der Ansprüche 9 oder 10, **dadurch gekennzeichnet, daß** Schritt a) in einem Mischergranulator durchgeführt wird, wobei das/die Alkalisierungsmittel in fester Form vorgelegt und einer Lösung, Dispersion und/oder Schmelze des/der Beschichtungsmaterialien beaufschlagt wird/werden.

12. Verfahren nach einem der Ansprüche 9 bis 11, **dadurch gekennzeichnet, daß** die Beschichtung des/der Alkalisierungsmittel in einer Wirbelschichtapparatur durchgeführt werden.

13. Verfahren nach einem der Ansprüche 9 bis 12, **dadurch gekennzeichnet, daß** das/die Alkalisierungsmittel mit einer Lösung oder Dispersion von Fettsäuren ausgewählt aus Caprylsäure. Pelargonsäure, Caprinsäure, Undecansäure, Laurinsäure, Tridecansäure, Tetradecansäure, Pentadecansäure, Palmitinsäure, Margarinsäure, Stearinsäure, Isostearinsäure, Nonadecansäure, Arachinsäure, Behensäure, Lignocerinsäure, Cerotinsäure, Melissinsäure, Crotonsäure, Ölsäure, Erucasäure, Sorbinsäure, Elaeostearinsäure, Docosahexaensäure besprüht und anschließend getrocknet wird/werden.

## Claims

1. An agent for lightening and/or colouring keratin fibres, in particular human hair, containing at least one solid alkalizing agent, **characterized in that** the alkalizing agent comprises a particle core, which contains alkalizing agent and comprises a shell surrounding said core, at least 50 wt.%, preferably at least 70 wt.%, more preferably at least 90 wt.% and in particular 100 wt.% of the weight of which shell consists of fatty acids selected from caprylic acid, pelargonic acid, capric acid, undecanoic acid, lauric acid, tridecanoic acid, tetradecanoic acid, pentadecanoic acid, palmitic acid, margaric acid, stearic acid, isostearic acid, nonadecanoic acid, arachidic acid, behenic acid, lignoceric acid, cerotic acid, melissic acid, crotonic acid, oleic acid, erucic acid, sorbic acid, elaeostearic acid, docosahexaenoic acid.

2. An agent according to claim 1, **characterized in that** the shell contains as fatty acids stearic acid and/or palmitic acid and/or oleic acid and/or isostearic acid or mixtures of these acids.

3. An agent according to either one of claims 1 or 2, **characterized in that** the shell consists of substances or substance mixtures which have a melting point or melting range between 30 and 100°C, preferably between 40 and 99°C and in particular between 50 and 95°C.

4. An agent according to any one of claims 1 to 3, **characterized in that** it additionally contains a solid peroxo compound, which is preferably selected from hydrogen peroxide addition compounds on solid carriers, ammonium and alkali metal persulfates and peroxydisulfates.

5. An agent according to claim 4, **characterized in that** it contains at least 2 different peroxydisulfates.

6. An agent according to any one of claims 1 to 5, **characterized in that** it contains at least one direct dye from the group comprising cationic (basic) dyes, preferably Basic Blue 6, C.I. no. 51,175; Basic Blue 7, C.I. no. 42,595; Basic Blue 9, C.I. no. 52,015; Basic Blue 26, C.I. no. 44,045; Basic Blue 41, C.I. no. 11,154; Basic Blue 99, C.I. no. 56,059; Basic Brown 4, C.I. no. 21,010; Basic Brown 16, C.I. no. 12,250; Basic Brown 17, C.I. no. 12,251; Basic Green 1, C.I. no. 42,040; Basic Orange 31; Basic Red 2, C.I. no. 50,240; Basic Red 22, C.I. no. 11,055; Basic Red 46; Basic Red 51; Basic Red 76, C.I. no. 12,245; Basic Violet 1, C.I. no. 42,535; Basic Violet 2; Basic Violet 3, C.I. no. 42,555; Basic Violet 10, C.I. no. 45,170; Basic Violet 14, C.I. no. 42,510; Basic Yellow 57, C.I. no. 12,719; Basic Yellow 87 and/or anionic (acidic) dyes, and/or nonionic dyes, preferably Acid Black 1, G.I. no. 20,470; Acid Black 52; Acid Blue 7; Acid Blue 9, C.I. no. 42,090; Acid Blue 74, C.I. no. 73,015, Acid Red 18, C.I. no. 16,255; Acid Red 23; Acid Red 27, C.I. no. 16,185; Acid Red 33; Acid Red 52; Acid Red 87, C.I. no. 45,380; Acid Red 92, C.I. no. 45,410; Acid Orange 3; Acid Orange 7; Acid Violet 43, C.I. no. 60,730; Acid Yellow 1, C.I. no. 10,316; Acid Yellow 10; Acid Yellow 23, C.I. no. 19,140; Acid Yellow 3, C.I. no. 47,005; Acid Yellow 36; D&C Brown no. 1, C.I. no. 20,170 (Acid Orange 24); D&C Green no. 5, C.I. no. 61,570 (Acid Green G); D&C Orange no. 4, C.I. no. 15,510 (Acid Orange II); D&C Orange no. 10, C.I. no. 45,425: 1 (Solvent Red 73); D&C Orange no. 11, C.I. no. 45,425 (Acid Red 95); D&C Red no. 21, G.I. no. 45,380: 2 (Solvent Red 43); D&C Red no. 27. C.I. no. 45,410: 1 (Solvent Red 48); D&C Red no. 33, C.I. no. 17,200 (Acid Red 2A, Acid Red B); D&C Yellow no. 7, C.I. no. 45,350: 1 (Solvent Yellow 94); D&C Yellow no. 8, C.I. no. 45,350 (Acid Yellow 73); FD&C Red no. 4, C.I. no. 14,700 (Food Red 4); FD&C Yellow no. 6, C.I. no. 15,985 (Food Yellow 3); Food Green 3; Pigment Red 57-1; Disperse Black 9; Disperse Blue 1; Disperse Blue 3; Disperse Violet 1; Disperse Violet 4; HC Orange 1; HC Red 1; HC Red 3; HC Red 13; HC Yellow 2; HC Yellow 4; Na picramate; 1,4-bis-(2'-hydroxyethyl)amino-2-nitro-p-phenylenediamine; HC Yellow 5; HC Blue 2; HC Blue 12; 4-amino-3-nitrophenol; HC Yellow 6; HC Yellow 12; 2-nitro-1-(2'-hydroxyethyl)amino-4-methylbenzene; 2-nitro-4-aminodiphenylamine-2-carboxylic acid; 2-amino-6-chloro-4-nitrophenol; HC Red BN; 6-nitro-1,2,3,4-tetranitroquinoxaline; o-nitro-p-phenylenediamine; p-nitro-m-phenylenediamine; HC Red B 54; HC Red 10; HC Red 11; HC Red 13; 2-(2'-hydroxyethyl)amino-1-hydroxy-4,6-dinitrobenzene; 4-ethylamino-3-nitrobenzoic acid; 2-chloro-6-ethylamino-4-nitrophenol; 2-hydroxy-1,4-napthoquinone; 1-propene-(4-amino-2-nitrophenyl)amine; isatin; N-methyl isatin; HC Violet 1; HC Violet 2; 4-nitrophenyl aminoethylurea in quantities of 0.01 to 25 wt.%, preferably of 0.5 to 10 wt.%, in particular of 1 to 5 wt.%, in each case relative to the total agent.

7. An agent according to any one of claims 1 to 6, **characterized in that** it contains at least one direct dye, selected from Basic Blue 7, Basic Blue 99, Basic Violet 14, Basic Brown 16, Basic Brown 17, Basic Orange 31, Basic Red 46, Basic Red 51, Basic Red 76, Basic Yellow 57, Basic Yellow 87, Acid Black 1, Acid Blue 7, Acid Violet 43, Acid Red 23, Acid Red 52, Acid Orange 7, Acid Yellow 1, Acid Yellow 10, Acid Yellow 36, Food Green 3, Pigment Red 57-1, Disperse Black 9, Disperse Blue 1, Disperse Blue 3, Disperse Violet 1, Disperse Violet 4, HC Orange 1, HC Red 1, HC Red 3, HC Red 13, HC Yellow 2, HC Yellow 4, Na picramate, 1,4-bis-(2'-hydroxyethyl)amino-2-nitro-p-phenylenediamine, HC Yellow 5, HC Blue 2, HC Blue 12, 4-amino-3-nitrophenol, HC Yellow 6, HC Yellow 12, 2-nitro-1-(2'-hydroxyethyl)amino-4-methylbenzene, 2-nitro-4-aminodiphenylamine-2-carboxylic acid, 2-amino-6-chloro-4-nitrophenol, HC Red BN; 6-nitro-1,2,3,4-tetranitroquinoxaline, o-nitro-p-phenylenediamine, p-nitro-m-phenylenediamine, HC Red B 54, HC Red 10, HC Red 11, HC Red 13, 2-(2'-hydroxyethyl)amino-1-hydroxy-4,6-dinitrobenzene, 4-ethylamino-3-nitrobenzoic acid, 2-chloro-6-ethylamino-4-nitrophenol, 2-hydroxy-1,4-napthoquinone, 1-propene-(4-amino-2-nitrophenyl)amine, isatin, N-methyl isatin, HC Violet 1, HC Violet 2, 4-nitrophenyl aminoethylurea in quantities of 0.01 to 25 wt.%, preferably of 0.5 to 10 wt.%, in particular of 1 to 5 wt.%, in each case relative to the total agent.

8. An agent according to any one of claims 1 to 7, **characterized in that** it contains at least one dye precursor from the groups comprising aromatic and heteroaromatic diamines, aminophenols, naphthols, polyphenols, CH-acidic coupler components and the derivatives thereof in quantities of 0.01 to 25 wt.%, preferably of 0.5 to 10 wt.%, in particular of 1 to 5 wt.%, in each case relative to the total agent.

9. A method of producing agents for lightening and/or colouring keratin fibres, **characterized by** the steps of
a) coating at least one alkalizing agent with a coating composition to form a shell, at least 50 wt.%, preferably at least 70 wt.%, more preferably at least 90 wt.% and in particular 100 wt.% of the weight of which shell consists of fatty acids selected from caprylic acid, pelargonic acid, capric acid, undecanoic acid, lauric acid, tridecanoic acid, tetradecanoic acid, pentadecanoic acid, palmitic acid, margaric acid, stearic acid, isostearic acid, nonadecanoic acid, arachidic acid, behenic acid, lignoceric acid, cerotic acid, melissic acid, crotonic acid, oleic acid, erucic acid, sorbic acid, elaeostearic acid, docosahexaenoic acid,
b) mixing the coated alkalizing agent(s) with further ingredients of bleaching and/or colouring agents for keratin fibres.

10. A method according to claim 9, **characterized in that** the coated alkalizing agent (including coating) contains, relative to its weight, 5 to 80 wt.%, preferably 10 to 75 wt.% and in particular 20 to 70 wt.% of alkalizing agent.

11. A method according to either one of claims 9 or 10, **characterized in that** step a) is performed in a mixer/granulator, the alkalizing agent(s) being initially introduced in solid form and exposed to a solution, dispersion and/or melt of the coating material(s).

12. A method according to any one of claims 9 to 11, **characterized in that** coating of the alkalizing agent(s) is performed in a fluidized bed apparatus.

13. A method according to any one of claims 9 to 12, **characterized in that** the alkalizing agent(s) is/are sprayed with a solution or dispersion of fatty acids selected from caprylic acid, pelargonic acid, capric acid, undecanoic acid, lauric acid, tridecanoic acid, tetradecanoic acid, pentadecanoic acid, palmitic acid, margaric acid, stearic acid, isostearic acid, nonadecanoic acid, arachidic acid, behenic acid, lignoceric acid, cerotic acid, melissic acid, crotonic acid, oleic acid, erucic acid, sorbic acid, elaeostearic acid, docosahexaenoic acid and is/are then dried.

## Revendications

1. Agent pour l'éclaircissement et/ou la teinture de fibres kératiniques, en particulier de cheveux humains, contenant au moins un agent d'alcalinisation solide, **caractérisé en ce que** l'agent d'alcalinisation comprend un noyau particulaire qui contient un agent d'alcalinisation et une enveloppe entourant ce noyau, qui est constituée à concurrence d'au moins 50 % en poids, de préférence à concurrence d'au moins 70 % en poids, de manière plus préférée d'au moins 90 % en poids et en particulier à concurrence de 100 % en poids de son poids par des acides gras choisis parmi l'acide caprylique, l'acide pélargonique, l'acide caprique, l'acide undécanoïque, l'acide laurique, l'acide tridécanoïque, l'acide tétradécanoïque, l'acide pentadécanoïque, l'acide palmitique, l'acide margarinique, l'acide stéarique, l'acide isostéarique, l'acide nonadécanoïque, l'acide arachidique, l'acide béhénique, l'acide lignocérique, l'acide cérotique, l'acide mélissique, l'acide crotonique, l'acide oléique, l'acide érucique, l'acide sorbique, l'acide élaéostéarique, l'acide docosahexaénoïque.

2. Agent selon la revendication 1 **caractérisé en ce que** l'enveloppe contient, à titre d'acide gras, de l'acide stéarique et/ou de l'acide palmitique et/ou de l'acide oléique et/ou de l'acide isostéarique ou des mélanges de ces acides.

3. Agent selon l'une quelconque des revendications 1 ou 2 **caractérisé en ce que** l'enveloppe est constituée par des substances ou des mélanges de substances qui présentent un point de fusion ou une plage de fusion entre 30 et 100 °C, de préférence entre 40 et 99 °C et en particulier entre 50 et 95 °C.

4. Agent selon l'une quelconque des revendications 1 à 3 **caractérisé en ce qu'**il contient en outre un composé peroxo solide qui est de préférence choisi parmi des composés de fixation par addition de peroxyde d'hydrogène sur des supports solides, des persulfates et des peroxydisulfates d'ammonium et de métaux alcalins.

5. Agent selon la revendication 4 **caractérisé en ce qu'**il contient au moins 2 peroxydisulfates différents.

6. Agent selon l'une quelconque des revendications 1 à 5 **caractérisé en ce qu'**il contient au moins un colorant montant directement sur la fibre choisi parmi le groupe des colorants cationiques (basiques), de préférence Basic Blue 6, C.I. n° 51,175 ; Basic Blue 7, C.I. n° 42,595 ; Basic Blue 9, C.I. n° 52,015 ; Basic Blue 26, C.I. n° 44,045 ; Basic Blue 41, C.I. n° 11,154 ; Basic Blue 99, C.I. n° 56,059 ; Basic Brown 4, C.I. n° 21,010 ; Basic Brown 16, C.I. n° 12,250 ; Basic Brown 17, C.I. n° 12,251 ; Basic Green 1, C.I. n° 42,040 ; Basic Orange 31 ; Basic Red 2, C.I. n° 50,240 ; Basic Red 22, C.I. n° 11,055 ; Basic Red 46 ; Basic Red 51 ; Basic Red 76, G.I. n° 12,245 ; Basic Violet 1, C.I. n° 42,535 ; Basic Violet 2 ; Basic Violet 3. C.I. n° 42,555 ; Basic Violet 10, C.I. n° 45,170 ; Basic Violet 14, C.I. n° 42,510 ; Basic Yellow 57, C.I. n° 12,719 ; Basic Yellow 87 et/ou des colorants anioniques (acides), et/ou des colorants non ioniques, de préférence Acid Black 1, C.I. n° 20,470 ; Acid Black 52 ; Acid Blue 7 ; Acid Blue 9, C.I. n° 42,090 ; Acid Blue 74, G.I. n° 73,015 ; Acid Red 18 ; C.I. n° 16,255 ; Acid Red 23 ; Acid Red 27, C.I. n° 16,185 ; Acid Red 33 ; Acid Red 52 ; Acid Red 87 ; C.I. n° 45,380 ; Acid Red 92, C.I. n° 45,410 ; Acid Orange 3 ; Acid Orange 7 ; Acid Violet 43, C.I. n° 60,730 ; Acid Yellow 1, C.I. n° 10,316 ; Acid Yellow 10 ; Acid Yellow 23, C.I, n° 19,140 ; Acid Yellow 3, C.I. n° 47,005 ; Acid Yellow 36 ; D & C Brown n° 1, C.I. n° 20,170 (Acid Orange 24) ; D & C Green n° 5, C.I. n° 61,570 (Acid Green G) ; D & C Orange n° 4, C.I. n° 15,510 (Acid Orange II) ; D & C Orange n° 10, C.I. n° 45,425 : 1 (Solvent Red 73) ; D & C Orange n° 11, C,I. n° 45,425 (Acid Red 95) ; D & C Red n° 21, C.I. n° 45,380 : 2 (Solvent Red 43) ; D & C Red n° 27. C.I. n° 45,410 : 1 (Solvent Red 48) ; D & C Red n° 33. C.I. n° 17,200 (Acid Red 2A, Acid Red B) ; D & C Yellow n° 7, C.I. n° 45,350 : 1 (Solvent Yellow 94) ; D & C Yellow n° 8, C.I. n° 45,350 (Acid Yellow 73) ; FD & C Red n° 4, C.I. n° 14,700 (Food Red 4) ; FD & C Yellow n° 6, C.I. n° 15,985 (Food Yellow 3) ; Food Green 3 ; Pigment Red 57-1 ; Disperse Black 9 ; Disperse Blue 1 ; Disperse Blue 3 ; Disperse Violet 1 ; Disperse Violet 4 ; HC Orange 1 ; HC Red 1 ; HC Red 3 ; HC Red 13 ; HC Yellow 2 ; HC Yellow 4 ; le picramate de sodium ; la 1,4-bis-(2'-hydroxyéthyl)amino-2-nitro-p-phénylènediamine ; HC Yellow 5 ; HC Blue 2 ; HC Blue 12 ; le 4-amino-3-nitrophénol ; HC Yellow 6 ; HC Yellow 12 ; le 2-nitro-1-(2'-hydroxyéthyl)amino-4-méthylbenzène ; l'acide 2-nitro-4-amino-diphénylamine-2-carboxylique ; le 2-amino-6-chloro-4-nitrophénol ; HC Red BN ; la 6-nitro-1,2,3,4-tétranitroquinoxaline ; la o-nitro-p-phénylènediamine ; la p-nitro-m-phénylénediamine ; HC Red B 54 ; HC Red 10 ; HC Red 11 ; HC Red 13 ; le 2-(2'-hydroxyéthyl)amino-1-hydroxy-4,6-dinitrobenzène ; l'acide 4-éthylamino-3-nitrobenzoïque ; le 2-chloro-6-éthylamino-4-nitrophénol; la 2-hydroxy-1,4-naphtoquinone ; la 1-propén-(4-amino-2-nitrophényl)amine ; l'isatine ; la N-méthyllisatine ; HC Violet 1 ; HC Violet 2 ; la 4-nitrophényl-aminoéthylurée dans des quantités de 0,01 à 25 % en poids, de préférence de 0,5 à 10 % en poids, en particulier de 1 à 5 % en poids, chaque fois rapportés à l'agent dans sa totalité.

7. Agent selon l'une quelconque des revendications 1 à 6, **caractérisé en ce qu'**il contient au moins un colorant montant directement sur la fibre choisi parmi Basic Blue 7, Basic Blue 99, Basic Violet 14, Basic Brown 16, Basic Brown 17, Basic Orange 31, Basic Red 46, Basic Red 51, Basic Red 76, Basic Yellow 57, Basic Yellow 87, Acid Black 1, Acid Blue 7, Acid Violet 43, Acid Red 23, Acid Red 52, Acid Orange 7, Acid Yellow 1, Acid Yellow 10, Acid Yellow 36, Food Green 3, Pigment Red 57-1, Disperse Black 9, Disperse Blue 1, Disperse Blue 3, Disperse Violet 1, Disperse Violet 4, HC Orange 1, HC Red 1, HC Red 3, HC Red 13, HC Yellow 2, HC Yellow 4, le picramate de sodium ; la 1,4-bis-(2'-hydroxyéthyl)amino-2-nitro-p-phénylènediamine; HC Yellow 5, HC Blue 2, HC Blue 12, le 4-amino-3-nitrophénol, HC Yellow 6, HC Yellow 12, le 2-nitro-1-(2'-hydroxyéthyl)amino-4-méthylbenzéne, l'acide 2-nitro-4-amino-diphénylamin-2-carboxylique, le 2-amino-6-chloro-4-nitrophénol, HC Red BN , la 6-nitro-1,2,3,4-tétranitroquinoxaline, la o-nitro-p-phénylènediamine, la p-nitro-m-phénylènediamine, HC Red B 54, HC Red 10, HC Red 11, HC Red 13, le 2-(2'-hydroxyéthyl)amino-1-hydroxy-4,6-dinitrobenzéne, l'acide 4-éthylamino-3-nitrobenzoïque, le 2-chlor-6-éthylamino-4-nitrophénol, la 2-hydroxy-1,4-naphtoquinone, la 1-propén-(4-amino-2-nitrophényl)amine, l'isatine, la N-méthylisatine, HC Violet 1, HC Violet 2, la 4-nitrophényl-aminoéthylurée dans des quantités de 0,01 à 25 % en poids, de préférence de 0,5 à 10 % en poids, en particulier de 1 à 5 % en poids, chaque fois rapportés à l'agent dans sa totalité.

8. Agent selon l'une quelconque des revendications 1 à 7, **caractérisé en ce qu'**il contient au moins un précurseur de colorant choisi parmi les groupes des diamines aromatiques et hétéroaromatiques, des aminophénols, des naphtols, des polyphénols, des composants faisant office de coupleurs contenant un groupe C-H acide, et de leurs dérivés, dans des quantités de 0,01 à 25 % en poids, de préférence de 0,5 à 10 % en poids, en particulier de 1 à 5 % en poids, chaque fois rapportés à l'agent dans sa totalité.

9. Procédé pour la préparation d'agents pour l'éclaircissement et/ou la teinture de fibres kératiniques, **caractérisé par** les étapes :
a) d'enduction d'au moins un agent d'alcalinisation avec un agent d'enduction pour obtenir une enveloppe qui est constituée à concurrence d'au moins 50 % en poids, de préférence à concurrence d'au moins 70 % en poids, de manière plus préférée d'au moins 90 % en poids et en particulier à concurrence de 100 % en poids de son poids par des acides gras choisis parmi l'acide caprylique, l'acide pélargonique, l'acide caprique, l'acide undécanoïque, l'acide laurique, l'acide tridécanoïque, l'acide tétradécanoique, l'acide pentadécanoïque, l'acide palmitique, l'acide margarinique, l'acide stéarigue, l'acide isostéarique, l'acide nonadécanoïque, l'acide arachidique, l'acide béhénique, l'acide lignocérique, l'acide cérotique, l'acide mélissique, l'acide crotonique, l'acide oléique, l'acide érucique, l'acide sorbique, l'acide élaéostéarique, l'acide docosahexaénoïque ;
b) de mélange du/des agents d'alcalinisation enduit(s) avec d'autres constituants d'agents de décoloration et/ou de teinture pour des fibres kératiniques.

10. Procédé selon la revendication 9, **caractérisé en ce que** l'agent d'alcalinisation enduit (y compris l'enduction), contient, rapportés à son poids, un agent d'alcalinisation à concurrence de 5 à 80 % en poids, de préférence à concurrence de 10 à 75 % en poids et en particulier à concurrence de 20 à 70 % en poids.

11. Procédé selon l'une quelconque des revendications 9 ou 10, **caractérisé en ce qu'**on met en oeuvre l'étape a) dans un mélangeur/granulateur, l'agent/les agents d'alcalinisation étant déposé(s) au préalable sous forme solide, avant d'être soumis à l'action d'une solution, d'une dispersion et/ou d'une masse fondue de la/des matière(s) d'enduction.

12. Procédé selon l'une quelconque des revendications 9 à 11, **caractérisé en ce que** l'enduction de l'agent/des agents d'alcalinisation est mise en oeuvre dans un appareil du type à lit fluidisé.

13. Procédé selon l'une quelconque des revendications 9 à 12, **caractérisé en ce qu'**on asperge l'agent/les agents d'alcalinisation avec une solution ou avec une dispersion d'acides gras choisis parmi l'acide caprylique, l'acide pélargonique, l'acide caprique, l'acide undécanoïque, l'acide laurique, l'acide tridécanoïque, l'acide tétradécanoïque, l'acide pentadécanoïque, l'acide palmitique, l'acide margarinique, l'acide stéarique, l'acide isostéarique, l'acide nonadécanoïque, l'acide arachidique, l'acide béhénique, l'acide lignocérique, l'acide cérotique, l'acide mélissique, l'acide crotonique, l'acide oléique, l'acide érucique, l'acide sorbique, l'acide élaéostéarique, l'acide docosahexaénoïque avant de procéder à un séchage.
